# EUROPEAN PATENT APPLICATION

(11) **EP 2 636 678 A1**
(43) Date of publication of application: **11.09.2013**
(21) Application number: 11837609.4
(22) Date of filing: 26.10.2011
(51) Int. Cl.: C07H 21/02, C12N 15/28, A61K 48/00, A61P 29/00

(54) **DERIVATIVES OF SMALL INTERFERING RNAS AND USE THEREOF**

(30) Priority: 04.11.2010 ES 201031622 P
(71) Applicant: Consejo Superior De Investigaciones Científicas (CSIC), 28006 Madrid (ES); Universidad Autónoma de Barcelona, 08193 Cerdanyola del Valles, Barcelona (ES); Universidad De Barcelona, 08028 Barcelona (ES)
(72) Inventor: OCAMPO, Sandra Milena, E-08034 Barcelona (ES); ERITJA CASADELLÁ, Ramón, E-08034 Barcelona (ES); ROMERO PRADA, Carolina, E-08193 Cerdanyola Del Valles (Barcelona) (ES); BURGUEÑO BANÚS, Juan Francisco, E-08193 Cerdanyola Del Valles (Barcelona) (ES); FERNÁNDEZ GIMENO, Ester, E-08193 Cerdanyola Del Valles (Barcelona) (ES); PERALES, José Carlos, E-08028 Barcelona (ES)
(74) Representative: Ungria López, Javier
(86) International application number: PCT/ES2011/070740
(87) International publication number: WO 2012/059612

(57) **Abstract**

The present invention relates to a small interfering RNA (siRNA) being 2'-O-methyl modified and there being attached thereto by a phosphodiester bond a position 3', wherein group R in position 3', wherein R is selected from among: a C₁-C₆ alkyl substituted with at least one -OH group; a C₄-C₆ cycloalkyl substituted with at least one -OH group; a C₄-C₆ heterocycloalkyl; a heteroalkyl chain having between 2 and 7 atoms, wherein at least one is an oxygen atom or a sulphur atom, the chain preferably being substituted with at least one -OH group; and a heteroaryl the ring whereof being formed of between 3 and 6 atoms and at least one of said atoms is an oxygen atom. The present invention furthermore refers to the use of said siRNA for the treatment of inflammatory diseases, preferably inflammatory bowel disease.

## Description

The present invention belongs to the field of Biotechnology. The present invention relates to new small interfering RNAs (siRNA) chemically modified at the ends of the partner strand and the use thereof in the treatment of diseases.

### STATE OF THE PRIOR ART

RNA interference is a post-transcriptional and sequence-specific gene silencing process, which uses double stranded RNA. After the discovery of this phenomenon in plants at the beginning of the 1990s, Andy Fire and Craig Mello demonstrated that a double-stranded RNA was able to specifically and selectively inhibit gene expression in *Caenorhabditis elegans* (Fire et al., 1998 Nature, 391 (6669) 806-11).

The sequence of the partner strand matches the corresponding region of the target messenger RNA (mRNA). The guide or leading strand is complementary to this mRNA. The double-stranded RNA is several orders of magnitude more efficient than the leading strand alone when it comes to silencing gene expression via the induction of mRNA degradation.

The RNA interference process begins when the enzyme DICER finds a double-stranded RNA and cuts it up in the so-called small interfering RNAs (siRNA, for "*small interfering RNAs*"), of about 20 nucleotides. The cells have a machinery called RISC ("*RNA-induced silencing complex*"), to which the guide strand is incorporated.

It is known that siRNA molecules can bind to "Toll"-like receptors present both in the cell membrane and in the interior of the cell, and stimulate the production of interferon alpha and beta which, in turn, produce an immune response. This immune response is non-specific and often masks the effects of the siRNAs.

The effect of some chemical changes in the siRNA molecules that decrease the immune response is described in the patent application WO2007051303. The modifications mentioned are found in both strands and are as follows: 2'-O-methyl, 2'-deoxi-2'-fluoro, 2'-deoxi and 2'-O-(2-methoxyethyl). Other more complex modifications are described in WO2006020768. In Van Aerschot et al, Bull. Soc. Chim. Belg. 104, n° 12, pp 717-720, 1995, a solid support functionalized with propanediol is used for the synthesis of DNA in order to increase the resistance to degradation by 3'-exonucleases.

As for the inflammatory bowel disease, some of the main features of the inflammatory bowel disease are loss of tolerance to intestinal microbes, the activation of the cells responsible for immunity and the production of large amounts of several cytokines such as tumour necrosis factor alpha, TNF-α and interleukins IL-1beta, IL-6 and IL-8 by immunocytes and also by the cells of the intestinal mucosa. These cytokines capture immunocompetent cells that amplify the inflammatory response. TNF-α plays a central role in the inflammatory process due to its capacity to increase its own expression at the same time that increases the expression of other proinflammatory molecules. Sera and intestinal mucosae of inflammatory bowel disease patients show high levels of TNF-α, and large amounts of TNF-α have been detected in inflammatory bowel disease models such as animal models of colitis. The inhibition of TNF-α through antibodies, soluble receptors or inhibitors of phosphodiesterase IV, such as rolipram, has significantly improved or even prevented inflammation in these animal models. However, several side effects such as for example an increase in vulnerability to intracellular pathogens or autoimmune reactions have been described. In order to reduce the side effects, it is suitable to increase the selectivity of the organ to which the treatment is addressed. In this regard, the patent application WO2006097768 describes the use of siRNAs able to inhibit the expression of IL-12 for the treatment of intestinal diseases and their administration by intrarectal route, and the application WO0020645 describes antisense oligonucleotides for inhibiting the expression of TNF-α. However these applications do not refer to possible chemical modifications for the reduction of the non-specific immune response.

The group of Dr. Ramratnam uses unmodified siRNAs (Zhang et al. Mol. Ther. 2006, 14: 336-42) and demonstrates that these siRNAs can reduce the levels of TNF-α mRNA and improve its histological profile but not the clinical development of the disease.

Thus, it is important to develop new strategies that allow, on the one hand, reducing the side effects associated with the treatments with siRNA and, on the other hand, directing the treatment to areas that you wish to treat, such as it is only the intestine in the inflammatory bowel disease.

### DESCRIPTION OF THE INVENTION

The present invention relates to compounds derived from small interfering RNAs (siRNAs) based on the 2'-O-methyl chemical modifications and on the binding of a small alkyl polar molecule, conferring surprisingly advantageous properties to the RNA duplex to silence gene expression without causing inflammatory side effects. For example, the siRNAs of the invention are able to inhibit the expression of the tumour necrosis factor alpha (TNF-α) without any side effects of stimulation of the immune response and to improve clinical outcomes in a model of colitis.

The fact that the 2'-O-methyl chemical modifications in a siRNA decrease the immune response and inflammation is already described in the patent application WO2007051303. However, the siRNA derivatives of the invention, modified with a small alkyl polar molecule in addition to the 2'-O-methyl modifications, have a surprisingly much more beneficial effect when it comes to inhibiting gene expression without causing stimulation of the immune response. These two modifications of the siRNAs: 2-O-methyl and binding of a small alkyl molecule, when performed separately achieve much less effective effects. Moreover, the effect achieved by the combination of these two modifications is much higher than the sum of the individual effects, i.e., it has a beneficial and unexpected synergistic effect.

The compounds of the present invention increase the inhibitory efficacy of TNF-α in cell cultures and animals. In addition, these modifications to the siRNAs reduce non-specific immune stimulation in PBMC human cells, thus improving the pharmacological profile.

The present invention also relates to a composition comprising said modified siRNAs, as well as the use of the siRNAs or of said composition for the preparation of a medicine.

Therefore, a first aspect of the present invention is related to a compound (hereinafter called compound of the invention) of formula siRNA-P-O-R wherein:
i) siRNA is a small interfering RNA comprising at least one 2'-O-methyl modification;
ii) -P-O- represents a phosphodiester bond binding R to the 3' terminal position of any of the siRNA strands;
iii) wherein R is a radical which is selected from among: a C₁-C₆ alkyl substituted with at least one -OH group; a C₄-C₆ cycloalkyl substituted with at least one -OH group; a C₄-C₆ heterocycloalkyl; a heteroalkyl chain having between 2 and 7 atoms, wherein at least one of the atoms of the chain is an oxygen atom or a sulphur atom, the chain preferably being substituted with at least one -OH group; and a heteroaryl, the ring whereof being formed of between 3 and 6 atoms and at least one of said atoms is an oxygen atom.

The term small interfering RNA or siRNA in the present description is interchangeable with siRNA.

A siRNA is a double stranded RNA comprising two elements: (a) a guide strand, also known as leading strand or antisense strand that hybridizes to (b) a partner strand, also known as sense strand. The guide, leading or antisense strand is responsible for the ability of this molecule for interfering with the expression of messenger RNA (mRNA) which is the target of the silencing. Said guide strand hybridizes to said mRNA, finally causing its processing and thus reducing the translation of mRNA into protein.

A siRNA is formed by linked ribonucleotides, but it may also have some deoxyribonucleotide.

A 2'-O-methyl modification in a nucleotide is the binding of an oxy-methyl group in position 2' of the nucleotide, i.e. in the second carbon of the pentose.

The 3'-position is obviously the 3'-position of the terminal nucleotide, located at the 3' end of either of the two strands of the siRNA, since in the other nucleotides, the 3'-position is occupied by the bond to the next nucleotide of the strand by a phosphodiester bond.

A -OH group is a free hydroxyl group.

For the binding of R to the siRNA through a phosphodiester bond it is necessary that the group which will be incorporated has at least one free hydroxyl group, whose oxygen atom will form the bond with the siRNA. The phosphodiester bond can be such bond or any other bond that fulfils the same function, although preferably it is a phosphodiester bond.

In a preferred embodiment of the invention, the compound of the invention is anti-inflammatory. Preferably, the guide strand of the siRNA of the compound of the invention inhibits or silences, totally or partially, the expression of a pro-inflammatory molecule.

The term "inhibit or silence", as used in the present description is related to the reduction of gene expression that occurs when the siRNA specifically hybridizes to the mRNA to which it is aimed and induces its degradation. It is said that gene expression is inhibited or silenced when the amount of mRNA of a specific gene decreases or disappears with respect to the original starting levels. The amount or the levels of mRNA can be determined by multiple techniques known by the person skilled in the art, such as, but without limitation, the polymerase chain reaction (PCR) or other techniques of hybridization with specific probes.

In this description, proinflammatory molecules means those molecules that are involved in inflammation, promoting said process. There is a wide variety of proinflammatory molecules, which include interleukin 1 (IL-1), interleukin 8 (IL-8), prostaglandins, nitric oxide, interferon γ and tumour necrosis factor (TNF). There are also some inflammatory enzymes, like type II phospholipase or PLA₂, cyclooxygenase-2 (COX-2) or inducible nitric oxide synthase (iNOS), which activate the synthesis of the platelet activating factor, leukotrienes, prostanoids or nitric oxide.

In a preferred embodiment of the compound of the invention, the guide strand of the siRNA inhibits or silences, totally or partially, the expression of at least one of the genes of the list comprising: tumor necrosis factor alpha (TNF-α), IL-1 beta, IL-6 and IL-8. Preferably, the guide strand of the siRNA in the compound of the invention inhibits or silences, totally or partially, the expression of TNF-α gene or the IL-1 beta gene. More preferably, the guide strand of the siRNA of the compound of the invention inhibits or silences, totally or partially, the expression of the TNF-α gene.

The tumour necrosis factor or TNF-α is the first member of the superfamily of TNF, to which some cytokines involved in the acute phase of the inflammatory process belong. The members of this superfamily have a common phylogenetic origin and most of them also have a common function: they promote apoptosis and the activation of the NF- κB transcription factor.

In a preferred embodiment of the compound of the invention, R is a C₁-C₆ alkyl substituted with at least one free hydroxyl group. Preferably, R is a C₂-C₄ alkyl substituted with one or two free hydroxyl groups.

In a preferred embodiment of the compound of the invention, R is ethyl, propyl, isopropyl, n-butyl, isobutyl, or tert-butyl. Preferably, the phosphodiester bond binds a molecule of 1,2-propanediol or 1,3-propanediol, preferably 1,3-propanediol, in position 3' of any of the siRNA strands. Since a hydroxyl group is used for the formation of the bond, there would remain only one free hydroxyl group.

In a preferred embodiment of the compound of the invention, R is a C₄-C₆ cycloalkyl substituted with at least one free hydroxyl group. Preferably, R is cyclobutyl, cyclopentyl and cyclohexyl wherein R is substituted with one or two free hydroxyl groups. More preferably, the phosphodiester bond links a molecule of 4-cyclohexanediol in position 3' of any of the siRNA strands.

In another preferred embodiment of the compound of the invention, R is a C₄-C₆ heterocycloalkyl, preferably substituted with at least one free hydroxyl group. Preferably, R is an oxetanyl (C₄), aziridinyl (C₃), azetidinyl (C₄), tetrahydrofuranyl (C₅), pyrrolidinyl (C₅), morpholinyl (C₆), dithianyl (C₆), thiomorpholinyl (C₆), piperidinyl (C₆), tetrahydropyridinyl (C₆), piperazinyl (C₆) or trithianyl (C₆). More preferably, R is 4-methylpiperazinyl, 3-methyl-4-methylpiperazine, 4-dimethylaminopiperidinyl, 4-methylaminopiperidinyl, 4-aminopiperidinyl, 3-dimethylaminopiperidinyl, 3-methylaminopiperidinyl, 3-aminopiperidinyl, 4-hydroxypiperidinyl, 3-hydroxypiperidinyl, 2-hydroxypiperidinyl, 4-methylpiperidinyl, 3-methylpiperidinyl, 3-dimethylaminopyrrolidinyl, 3-methylaminopyrrolidinyl, 3-aminopyrrolidinyl or methylmorpholinyl.

In another preferred embodiment of the compound of the invention, R is a heteroalkyl chain having between 2 and 7 atoms, wherein at least one of the atoms of the chain is an oxygen atom or a sulphur atom, the chain preferably being substituted with at least one free hydroxyl group. Preferably, R is a heteroalkyl chain having between 4 and 6 atoms wherein at least one of the atoms of the chain is an oxygen atom or a sulphur atom, the chain preferably being substituted with at least one free hydroxyl group. More preferably, the phosphodiester bond binds a molecule of diethylene glycol or diethylene glycol thioether in position 3' of any of the siRNA strands.

In a preferred embodiment of the compound of the invention, R is a heteroaryl formed by between 3 to 6 atoms and at least one of those atoms is an oxygen atom. Preferably, R is thienyl, furanyl, pirrolyl, oxazolyl, thiazolyl, or imidazolyl.

In a preferred embodiment of the compound of the invention, R is substituted with two or three hydroxyl groups. Preferably, R is selected from the list comprising: 1,2-propanediol, 1,3-propanediol, 3-amino-1,2-propanediol, 2,2-dimethyl-1,3-propanediol, glycerol, ethylene glycol, diethylene glycol, diethylene glycol thioether, 1,6-hexanediol, cyclohexanediol.

In a preferred embodiment of the compound of the invention, R is attached through a phosphodiester bond to the 3' terminal position of the partner strand.

Modifications of the siRNA of the compound of the invention can be carried out by any of the methods known to date, including those described in the examples of the present specification, or as described in Vaishnaw, AK et *al.* 2010. Silence. 1:14.

In a preferred embodiment of the compound of the invention, between 1 and 7 nucleotides that form the siRNA, preferably of the partner strand, comprise a 2'-O-methyl modification. Preferably between 2 and 4 nucleotides, preferably of the partner strand, comprise a 2'-O-methyl modification. More preferably, 2 nucleotides, preferably of the partner strand, comprise a 2'-O-methyl modification.

In a preferred embodiment of the compound of the invention, the nucleotides that form the siRNA comprising the 2'-O-methyl modification are at the 5' end. Preferably, the nucleotides that form the siRNA comprising 2'-O-methyl modifications are in the partner strand, and preferably at the 5' end of the partner strand.

In a preferred embodiment of the compound of the invention, at least one nucleotide that forms the siRNA, preferably of the partner strand, is a deoxyribonucleotide. Preferably at least one nucleotide of the 3' end of the siRNA, preferably of the partner strand, is a deoxyribonucleotide. More preferably, 2 nucleotides of the 3' end of the siRNA, preferably of the partner strand, are deoxyribonucleotides.

In a preferred embodiment of the compound of the invention, the deoxyribonucleotides of the 3' end of the siRNA are thymidine deoxyribonucleotides (dT). Preferably, the deoxyribonucleotides are at the 3' end of the partner strand of the siRNA.

In a preferred embodiment of the compound of the invention, each siRNA strand comprises between 15 and 40 nucleotides. Preferably, each strand comprises between 17 and 30 nucleotides. More preferably, each strand comprises between 18 and 25 nucleotides.

In a preferred embodiment of the compound of the invention, the sequence of the siRNA partner strand is SEQ ID NO: 1. In a preferred embodiment of the compound of the invention, the sequence of the siRNA guide strand is SEQ ID NO: 2. In a preferred embodiment of the compound of the invention, the sequence of the siRNA partner strand is SEQ ID NO: 1 and the sequence of the siRNA guide strand is SEQ ID NO: 2.

The synthesis of the compound of the invention can be carried out according to any of the methods described in the prior art, or as described in Reese CB. Organic Biomolecular Chemistry. 2005, 3: 3851-68.

A second aspect of the present invention is related to a pharmaceutical composition characterized in that it comprises the compound of the invention, hereinafter called pharmaceutical composition of the invention.

In a preferred embodiment of the second aspect of the invention, the pharmaceutical composition comprises a pharmaceutically acceptable vehicle or diluent.

The "vehicle" or carrier is preferably an inert substance. The function of the vehicle is to facilitate the incorporation of other compounds, to allow better dosage and administration or to give consistency and form to the pharmaceutical composition. Therefore, the vehicle is a substance used in the medicine to dilute any of the components of the pharmaceutical composition of the present invention to a given volume or weight; or that even without diluting such components is able to allow better dosage and administration or to give consistency and form to the medicine. When the form of presentation is liquid, the pharmaceutically acceptable vehicle is the diluent.

In a preferred embodiment of the second aspect of the invention, the vehicle or diluent is a transfection agent. Preferably, the transfection agent is selected from the list comprising polyethylene glycol, cholesterol, polyethylenimide (PEI), cell penetrating peptides, oligoarginine, poly-lysine, rabies virus glycoprotein, gold nanoparticles, dendrimers, carbon nanotubes and lipids.

A transfection agent is any system that allows or facilitates the transfection, i.e. the introduction of a nucleic acid in a cell. In the present description, a transfection agent is a molecule that facilitates or enables the entry of the siRNAs of the invention or the composition comprising it, in a cell.

Preferably, the transfection agent is a lipid. More preferably, the lipid is a cationic lipid. Even more preferably, the cationic lipid is a liposome.

In another even more preferred embodiment, the pharmaceutical composition also comprises another biologically active substance. In addition to the requirement for the therapeutic efficacy, which may require the use of other therapeutic agents, there may be additional reasons that require or largely recommend using a combination of a compound of the invention and another therapeutic agent. The term "biologically active substance" is all matter, regardless of its source, whether it is human, animal, plant, chemical or otherwise, which has a proper activity for constituting a medicine.

A third aspect of the present invention is related to the use of the compound or the pharmaceutical composition of the invention for the preparation of a medicine.

In a preferred embodiment of the third aspect of the invention, the compound or the pharmaceutical composition of the invention are used for the treatment of an inflammatory disease. Preferably, the inflammatory disease is selected from the list comprising: inflammatory bowel disease, rheumatoid arthritis, osteoarthritis and chronic obstructive pulmonary disease. More preferably, the inflammatory disease is intestinal; preferably it is inflammatory bowel disease.

The compound or pharmaceutical composition of the invention can be used for the treatment of Crohn's disease, ulcerative colitis, irritable bowel syndrome, infectious bowel disease, pseudomembranous colitis, amebiasis, intestinal tuberculosis, chronic polyps, diverticular disease, constipation, intestinal obstruction, malabsorption syndrome, diarrhea or colorectal cancer.

In a preferred embodiment of the third aspect of the invention, the compound or the pharmaceutical composition of the invention are used for the treatment of a disease that is selected from the list comprising: Crohn's disease, ulcerative colitis, irritable bowel syndrome, infectious bowel disease, pseudomembranous colitis, amebiasis or intestinal tuberculosis, chronic polyps, diverticular disease and colorectal cancer. Preferably, the disease is Crohn's disease, ulcerative colitis or irritable bowel syndrome.

In a preferred embodiment of the third aspect of the invention, the compound or the pharmaceutical composition containing it is administered by intrarectal route. For said administration there can be used, without limitation, suppositories or enemas rectally.

In a preferred embodiment of the third aspect of the invention, a therapeutically effective amount of the compound or pharmaceutical composition of the invention is administered. The expression "therapeutically effective amount" means an amount that, administered in doses and during the required time period, is effective in achieving the desired prophylactic or therapeutic result. A "therapeutically effective amount" of the compound or the pharmaceutical composition of the invention may vary with the stage of the disease, age, sex and weight of the individual, and is related to an amount that does not have adverse effects or toxicity and is able to achieve the desired therapeutic or prophylactic effect.

One embodiment of the invention can be a compound in which each strand of the siRNA has between 17 and 30 nucleotides, comprises between two and four 2'-O-methyl modifications in the nucleotides of the 5' end of the partner strand, one modification with a 1,2-propanediol in the 3' end of the partner strand linked by a phosphodiester bond and the between two and four nucleotides of the 3' end of the partner strand are deoxyribonucleotides.

Another embodiment of the invention can be a pharmaceutical composition comprising a compound that inhibits or silences the expression of IL-1 in which each strand of the siRNA has between 20 and 26 nucleotides, comprises between two to five 2'-O-methyl modifications in the nucleotides of the partner strand and one modification with a C₂-C₄ alkyl substituted with two hydroxyl groups, linked to the partner strand at the 3' end by a phosphodiester bond and between two and four nucleotides of the 3' end of the partner strand are thymidine deoxyribonucleotides.

Another embodiment of the invention can be the use of a compound that inhibits the expression of a pro-inflammatory molecule comprising between two and four 2'-O-methyl modifications in between one and three nucleotides of the 3' end of any of the strands and one modification with 1,3-propanediol, for the preparation of a medicine for the treatment of inflammatory bowel disease that is administered by intrarectal route in a therapeutically effective amount.

The methods of synthesis of the compounds of the invention are known and they are available in the art. (Reese CB. Organic Biomolecular Chemistry. 2005, 3: 3851-68).

For example, the following scheme summarizes the synthesis of the glass support for the synthesis of RNA derivatives containing propanediol groups at the 3' end. 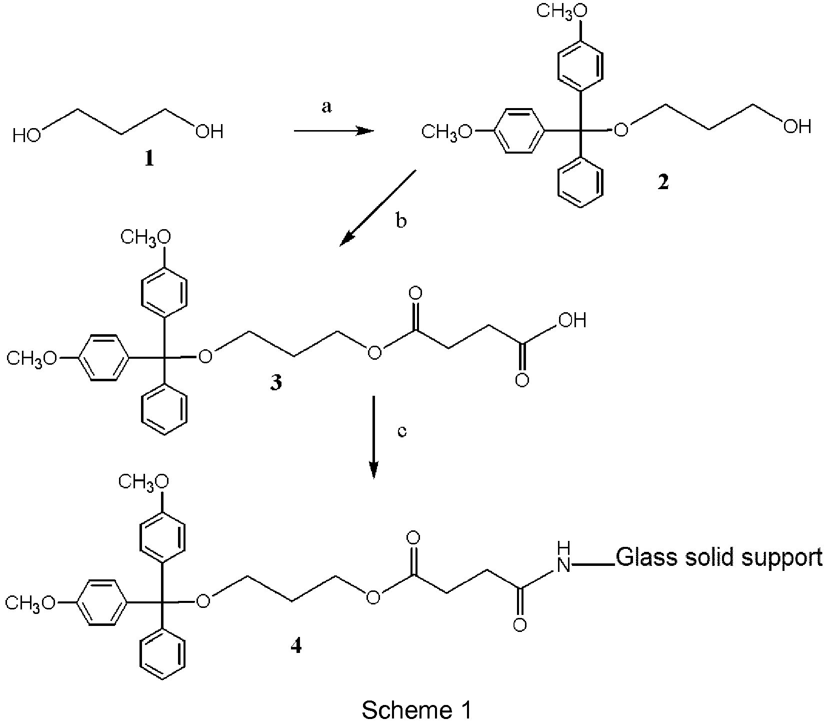 (1) is 1,3-propanediol; (a) the reagent used in this reaction is dimethoxytrityl chloride (DMT) dissolved in pyridine; (2) is 1-O-Dimethoxytrityl-1,3-propanediol; (3) is the hemisuccinate of 1-O-Dimethoxytrityl-1,3-propanediol, (b) the reagents used in this reaction are succinic anhydride, and N,N-dimethylaminopyridine dissolved in dichloromethane; (4) is the glass solid support functionalized with 1,3-propanediol, (c) the reagents used in this reaction are glass beads functionalized with amino, N,N-dimethylaminopyridine, triphenylphosphine, and 2,2-dithiobis (5-nitropyridine) groups dissolved in a mixture of 1,2-dichloroethane and acetonitrile.

**Table 1. Nomenclature of an embodiment of the invention (OMe-Prop) and controls used (w.m.: without modification, Scr or Scr OMe: random sequence siRNA being 2'-O-methyl modified; OMe: being 2'-O-methyl modified at the 5' end of the partner strand; Prop: with a molecule of 1,3-propanediol attached by a phosphodiester bond to the 3' end of the partner strand).**

| Compound | Guide strand Sequence | Partner strand Sequence | Number of 2'-O-methyl modifications | R |
|---|---|---|---|---|
| w.m. | SEQ ID NO: 5 | SEO ID NO: 2 | 0 | - |
| Scr* | SEQ ID NO: 4 | SEQ ID NO: 3 | 2 | - |
| OMe | SEQ ID NO: 5 | SEQ ID NO: 1 | 2 | - |
| Prop | SEQ ID NO: 5 | SEQ ID NO: 2 | 0 | 1,3-propanediol |
| OMe-Prop | SEQ ID NO: 5 | SEQ ID NO: 1 | 2 | 1,3-propanediol |

| | | | | |
|---|---|---|---|---|
| * SCR also can be found in the specification as Scr OMe. | | | | |

Throughout the description and claims the word "comprises" and its variants do not intend to exclude other technical features, additives, components or steps. For those skilled in the art, other objects, advantages and features of the invention will emerge partly from the description and partly from the practice of the invention. The following examples and drawings are provided by way of illustration and are not intended to be limiting of the present invention.

### DESCRIPTION OF THE FIGURES

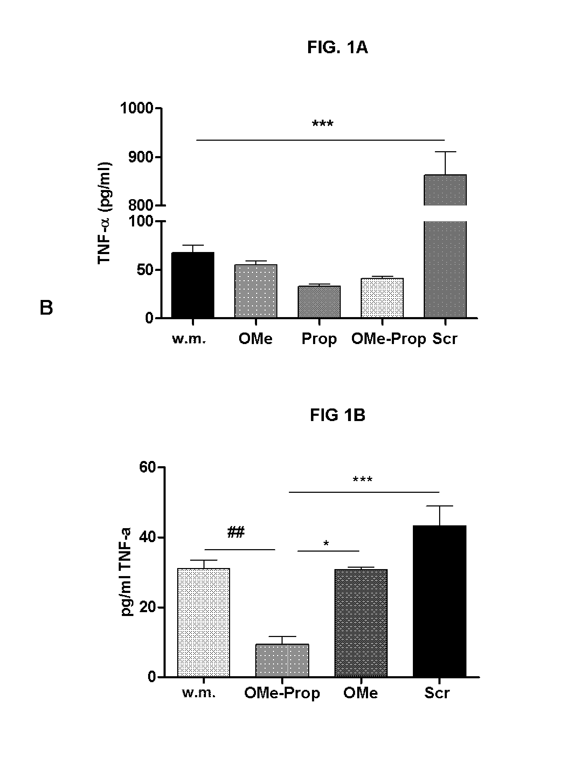

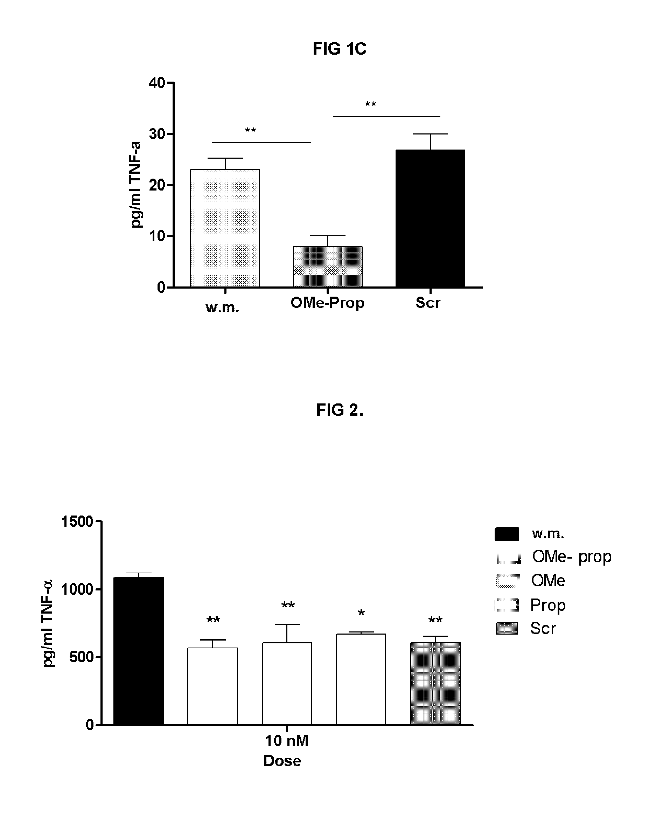

**Fig. 1****. Inhibitory activity of mouse TNF-a *in vitro* of the compounds of the invention and their controls in cell lines and primary macrophages.** Only the compounds wherein the sequence of the siRNA guide strand is complementary to the TNF-α mRNA (w.m., OMe, Prop, OMe-Prop) are able to bind RISC and silence TNF-α, while the random sequence siRNA being 2'-O-methyl modified does not inhibit the production of TNF-α (Scr). **A**. Amount of TNF-α produced by HeLa cells after 24 h treatment with the plasmid expressing the TNF-α and compounds of the invention or their controls. **B**. Amount of TNF-α produced by peritoneal macrophages transfected with the compounds of the invention and their controls. **C**. Amount of TNF-α produced by 4T1 cells after 24 h treatment with the compounds of the invention and their controls. Error bars represent standard deviation of the mean. The statistical analysis was performed following the ANOVA method with Bonferroni post-test. *** P<0.001 **P<0.01 or *P<0.05 compared with Scr, the random sequence siRNA, ## P<0.01 compared with the siRNA without modification (w.m.).

**Fig. 2****. Stimulation of the immune response produced by the addition of compounds of the invention and their controls.** The presence of modifications 2'-O-methyl and/or propanediol decreases the immune response to the compounds, regardless of the sequence of the siRNA. Peripheral blood mononuclear cells (PBMC) were transfected with a 10 nM concentration of the different compounds of the invention and their controls and the amount of human TNF-α produced in the supernatants was measured. OMe-Prop stimulates the immune response less than w.m.. Error bars represent standard deviation of the mean. The statistical analysis was performed following the ANOVA method with Bonferroni post-test. **P<0.01 or *P<0.05 compared with w.m..

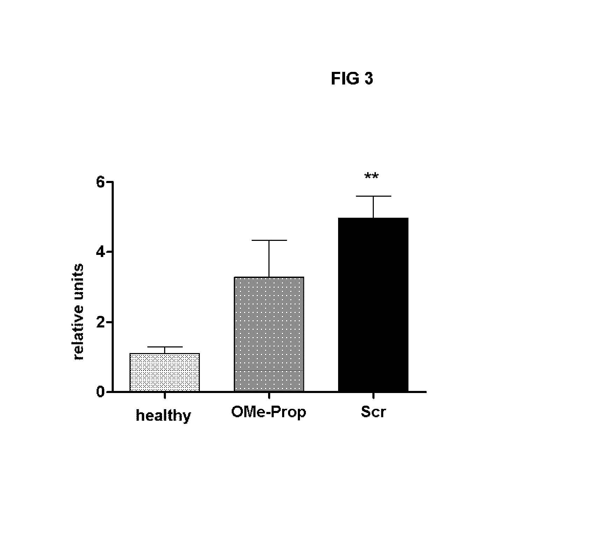

**Fig. 3****. Inhibition of TNF-α production in a model of chronic colitis.** It shows the levels of messenger RNA (mRNA) of TNF-α in arbitrary units in the colon of healthy mice or mice with colitis treated with OMe-Prop or Scr. The animals with colitis were treated with OMe-Prop or Scr. The colon was removed, mRNA was isolated and the levels of TNF-α were measured by quantitative PCR. The OMe-Prop compound is capable of reducing the production of TNF-α with respect to Scr. The statistical analysis was performed following the ANOVA method with Bonferroni post-test. **P<0.01.

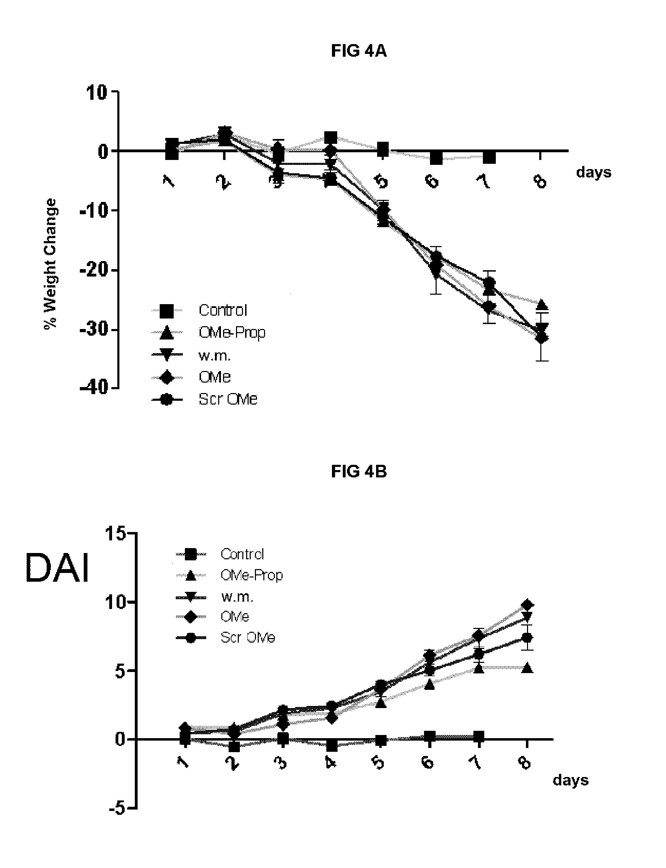

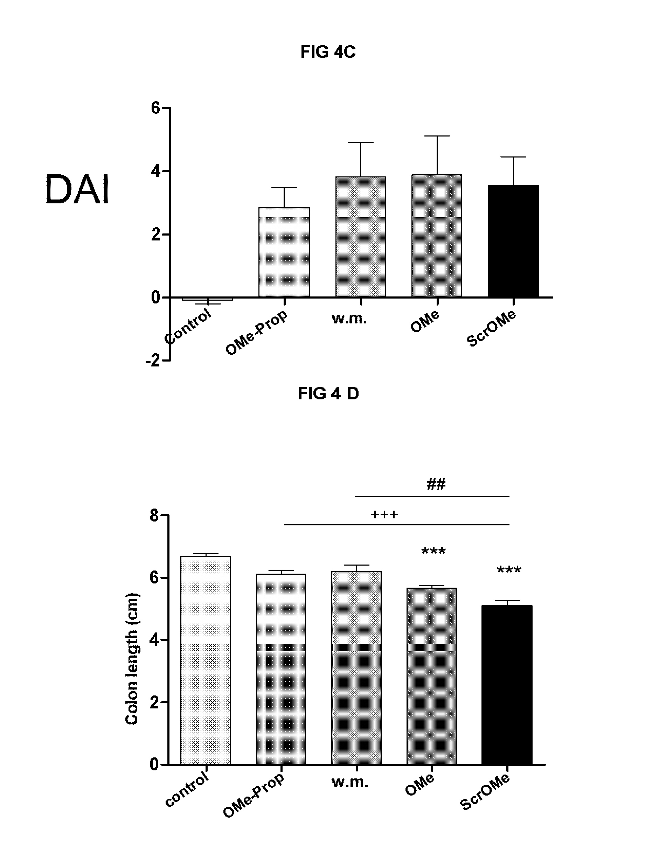

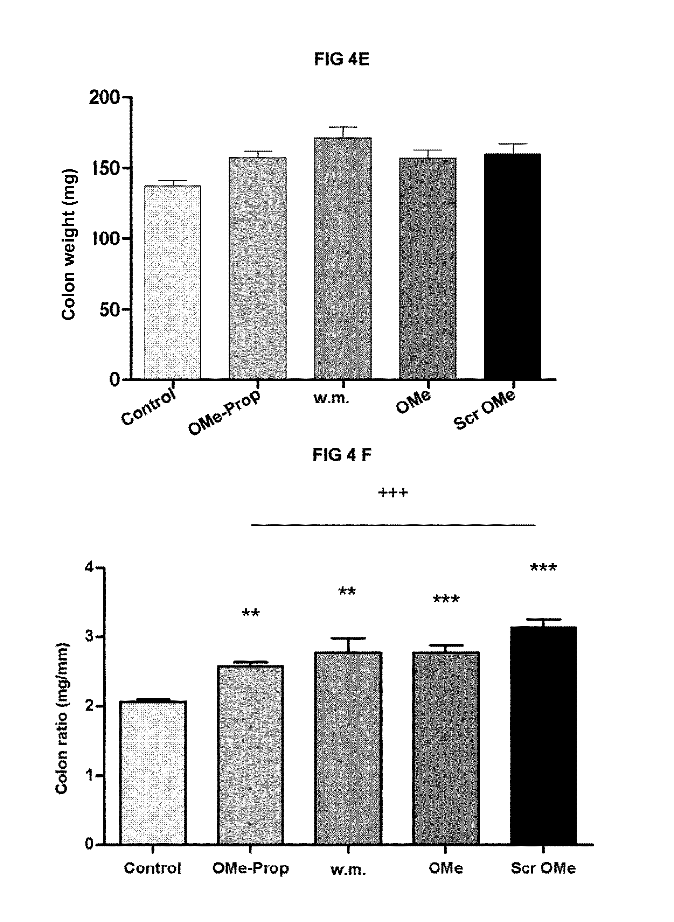

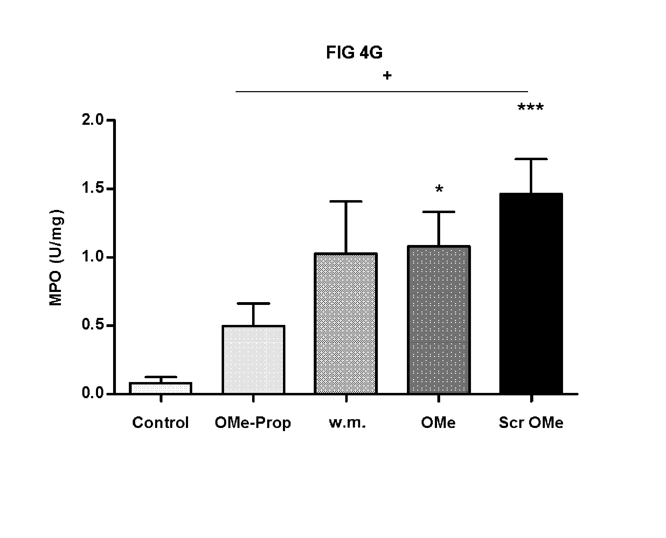

**Fig. 4****. Clinical outcome observed in a model of chronic colitis as a result of the administration of the compounds of the invention and their controls.** The compounds of the invention improve the clinical outcome in a model of chronic colitis. Animals with colitis were treated with OMe-Prop, w.m., OMe or Scr and were observed until day 8, during which days several disease indicators were measured and the disease activity index (DAI) was assessed. Mice were sacrificed 8 days after the beginning of the experiment and the colon was removed for evaluating the activity of myeloperoxidase (MPO) and histological observation. Control mice are healthy mice in which colitis was not induced. **A.** Observed changes in weight. **B.** Variation in the disease activity index (DAI). **C.** Mean of the disease activity indexes (DAI) of the animals treated at day 8. **D.** Mean of the length of the colon in cm in the animals treated at day 8. E. Mean of the weight of the colon in mg in the animals treated at day 8. F. Mean of the ratio between weight in mg and the length of the colon in mm of the animals treated at day 8. **G**. Mean of the levels of myeloperoxidase (MPO) in units per mg of colon determined in the colon of animals treated at day 8. The statistical analysis was performed following the ANOVA method with Bonferroni post-test. *** P<0.001 **P<0.01 or *P<0.05 compared to healthy controls, ## P<0.01 comparing w.m. to Scr OMe, +++ P<0.001 comparing OMe-Prop to Scr OMe.

### EXAMPLES

### EXAMPLE 1: Synthesis of the compounds.

RNA sequences were assembled using the method of synthesis in the solid phase. The partner strands carrying the propanediol group at the 3' end with or without the units of 2'-O-metil-RNA in the two final positions near the 5' end: 5'-(mG)(mU)GCCUAUGUCUCAGCCUC-dT-dT-propanediol-3' (SEQ ID NO: 1) and 5'-GUGCCUAUGUCUCAGCCUC-dT-dT-propanediol-3' (SEQ ID NO: 2) were assembled in a glass solid support following scheme 1.

### Preparation of solid support functionalized with 1,3-propanediol.

As shown in scheme 1, 1-O-dimethoxytrityl-1,3-propanediol (0.1 millimoles) prepared as described in Van Aerschot et al. Bull. Soc. Chim. Belg. 104, n° 12, pp 717-720, 1995; was dissolved in dichloromethane (DCM) (1 ml) along with N,N-dimethylaminopyridine (1.5 eq) and succinic anhydride (1.5 eq). The reaction was kept under stirring at room temperature for 16 hours (h). The resulting solution was diluted with 5 ml of DCM and the resulting organic solution was washed 2 times with a 0.1 M solution of Na₂HPO₄. The resulting organic layer was dried with anhydrous sodium sulfate, filtered and the solvent evaporated. The residue containing the desired hemisuccinate was used directly in the next step without further purification.

0.1 millimoles of 2,2-dithio-bis-(5-nitropyridine) (DTB) were dissolved in 0.4 ml of acetonitrile and the resulting solution was mixed with a solution containing 0.1 millimoles of the hemisuccinate and N,N-dimethylaminopyridine (0.1 millimoles) dissolved in 0.5 ml of acetonitrile (ACN). A mixture of 0.1 millimoles of triphenylphosphine (TPP) and 0.2 ml of ACN were added to the above solution at room temperature.

The mixture was stirred with an orbital shaker for a few seconds and added to a syringe containing 0.5 g of controlled pore glass beads functionalized with amino groups. The reaction was left stirring gently for 2 hours at room temperature. The reaction was stopped by adding 0.5 ml of methanol and the solid support was removed from the solution. The solid support was washed three times with methanol (10 ml per wash), three times with ACN (10 ml per wash) and three times with ethyl ether (10 ml per wash). The support was dried under vacuum and treated with the acetylation solution that consists of a mixture of two solutions: 1) 0.5 ml of a solution of acetic anhydride:pyridine:tetrahydrofuran (1:1:8) and 2) 0.5 ml of a 10% solution of N-methylimidazole in tetrahydrofuran. After 30 minutes, the solid support is washed as above with methanol, acetonitrile, and ethyl ether. The support was air dried and then under vacuum and kept at 4° C. The degree of functionalization was determined by elimination of the group dimethoxytrityl (DMT) of an aliquot with an acid solution and further reading of the absorption of the DMT cation released from the support at 500 nm. The degree of functionalization was 35 nanomoles/g.

### Synthesis of oligonucleotides

The oligoribonucleotides were prepared using a synthesizer from Applied Biosystems model 3400 using phosphoramidite of 2-cyanoethyl and the tert butyl dimethylsilyl-type protectors (TBDMS) for the protection of the hydroxyl group at position 2'. The following solutions were used: 0.4 M 1H-tetrazole in ACN (catalyst); 3% trichloroacetic acid in DCM (detritylation), acetic anhydride:pyridine:tetrahydrofuran (1:1:8) (acetylation solution A), 10% N-methylimidazole in tetrahydrofuran (acetylation solution B), 0.01 M iodine in tetrahydrofuran:pyridine:water (7:2:1) (oxidation). In the RNA sequences, the last DMT was eliminated since the DMT group is not completely stable to fluoride treatment. The average yield per stage of addition of one nucleotide was around 97-98% for monomers of RNA. The polymeric supports obtained were treated with a concentrated solution of ammonia-ethanol (3:1) for 1 h at 55° C. The supports were washed with ethanol and the resulting solutions were combined and evaporated to dryness. The resulting products were treated with 0.15 ml of triethylamine-tris(hydrofluoride):triethylamine:N-methylpyrrolidone (4:3:6) for 2.5 h at 65° C in order to eliminate the TBDMS groups. The reactions were stopped by addition of 0.3 ml isopropoxytrimethylsilane and 0.75 ml of ether. The resulting mixtures were stirred and cooled at 4° C. A precipitate formed which was centrifuged at 7,000 rpm for 5 minutes at 4° C. The precipitates were washed with ether and centrifuged again. The residues were dissolved in water and the conjugates were purified by HPLC. The column 120-10 Nucleosil C18 (250 x 4 mm) was used for HPLC. A linear gradient of 20 minutes from 0% to 50%; with a flow of 3 ml/min was used. The composition of the solutions used in HPLC was: solution A: 5% ACN in triethylammonium acetate 100 mM (pH 6.5) and solution B: 70% ACN in triethylammonium acetate 100 mM pH 6.5. The purified products were analyzed by MALDI-TOF mass spectrometry ("*Matrix-Assisted Laser Desorption*/*Ionization-Time of Flight*").

The MALDI-TOF spectra were performed in a Perseptive Voyager DETMRP mass spectrometer, equipped with a nitrogen laser at 337 nm using a 3 ns pulse. The used matrix contained 2,4,6-trihydroxyacetophenone (THAP, 10 mg/ml in ACN:water 1:1) and ammonium citrate (50 mg/ml in water).

The following RNA sequences were obtained from commercial sources (Sigma-Proligo, Dharmacon): negative control partner strand 5'-(mC)(mA)G UCG CGU UUG CGA CUG G-dT-dT-3'(SEQ ID NO: 3), negative control guide strand 5'-CCA GUC GCA AAC GCG ACU G-dT-dT-3' (SEQ ID NO: 4), anti-TNF-α guide strand: 5'-GAG GCU GAG ACA UAG GCA C-dT-dT-3' (SEQ ID NO: 5), anti-TNF-α partner strand: 5'-GUG CCU AUG UCU CAG CCU C-dT-dT-3' (SEQ ID NO: 2) and anti-TNF-α partner strand: 5'-(mG)(mU)G CCU AUG UCU CAG CCU C-dT-dT-3' (SEQ ID NO: 1). The RNA-type monomers (ribonucleotides) are detailed in capital letters and the abbreviation dT corresponds to the deoxyribonucleotide thymidine. The monomers of the type 2'-O-methyl-RNA are detailed in parentheses such as: (mG), (mU), (mC) and (mA). Anti-TNF-α siRNA sequences are described in Sorensen et al. J Mol Biol. 2003, 327: 761-6.

**Table 2. Characterization by mass spectrometry of modified RNA derivatives.**

| Type | Abbreviation | Experimental molecular weight | Theoretical molecular weight |
|---|---|---|---|
| Partner strand (SEQ ID NO: 1) modified with 3'-propanediol, 2'-O-Methyl | OMe-Prop | 6727 | 6725 |
| Partner strand (SEQ ID NO: 2) modified with 3'-propanediol | Prop | 6697 | 6697 |

### EXAMPLE 2: Inhibition of TNF-α in vitro.

Guide and partner strands were hybridized and the resulting double-stranded RNA was used for the inhibition of the expression of the TNF-α gene. First, we studied the inhibitory properties of the compounds prepared in example 1 in HeLa cells. These cells do not express the mouse TNF-α gene so, prior to the addition of the compounds, the cells were transfected with a plasmid containing the mouse TNF-α gene.

Figure 1 shows the biological effect of OMe, OMe-Prop and Prop. The silencing ability of these compounds was assessed both in cell lines (HeLa) and in primary cultures of macrophages. The results obtained with Hela cells using oligofectamine show that the compound of the invention OMe-Prop is able to bind RISC and to silence TNF-α (Fig. 1 A) while the random sequence siRNA (Scr) being 2'-O-methyl modified does not inhibit the production of TNF-α.

### Cell cultures, transfection, and cellular assays

HeLa cells were cultured in usual conditions: 37° C, CO₂ 5%, in Dulbecco's modified Eagle's medium, 10% fetal bovine serum (FBS), 2 mM L-glutamine supplemented with penicillin (100 U/ml) and streptomycin (100 mg/ml). All experiments were made with a cell confluence of 40-60%. HeLa cells were transfected with 250 ng of plasmid expressing the mouse TNF-α gene using lipofectin (Invitrogen) and following the instructions of the manufacturer.

Three different protocols for transfection were used according to the type of cells used:
1.- One hour after the transfection with plasmid expressing the mouse TNF-α gene, HeLa cells were transfected with 50 nM of compounds designed to inhibit TNF-α, using oligofectamine (Invitrogen). After 48 h the concentration of TNF-α was determined in cell culture supernatants using enzyme-linked immuno sorbent assay (ELISA) (Bender MedSystems) following the instructions of the manufacturer.
2. Peritoneal macrophages were isolated from untreated mice. The peritoneum of euthanized animals was injected with culture medium and the resulting liquid was collected and isolated. The cells obtained in this liquid were counted and seeded. This culture of peritoneal macrophages was transfected with the compounds using DOTAP (Roche) and following the instructions of the manufacturer. After 20 hours of transfection, the cells were stimulated with 10 ng/ml of lipopolysaccharide (LPS) for 10 hours and the amount of TNF-α produced was determined by the ELISA test.
3.- Mouse 4T1 cells were cultured under standard conditions. These cells express the mouse TNF-α gene in a natural way. Solutions containing 100 nM of each of the compounds and their controls and 10% fetal bovine serum were incubated a few minutes before their addition to the cells. After 24 h, the amount of TNF-α produced by the cells was determined by an ELISA-type test.

### Preparation of adherent peripheral blood mononuclear cells (PBMC)

The PBMC were obtained from human plasma using density gradient separation using Ficoll. The monocyte-enriched populations were isolated by adherence on plastic. After 3 hours of incubation at 37° C, the cells were transfected with the compounds and their controls for 18 hours using DOTAP (Roche) and following the instructions of the manufacturer. The cell supernatants were collected and the amount of TNF-α produced under immunostimulation was determined by ELISA.

To assess the side effects of transfection of compounds and their controls, regardless of the target gene that you want to silence, the transfection of the primary culture of macrophages was carried out with w.m., OMe, OMe-Prop and with Scr. As shown in Figure 1B, OMe-Prop shows a clear improvement in the inhibitory efficiency, increasing resistance to nucleases. This beneficial effect is reproduced again when OMe-Prop is used in 4T1 cells (Figure 1 C).

The modified siRNAs immunostimulation capacity was examined in PBMC from healthy humans. The siRNA designed to inhibit mouse TNF-α cannot inhibit the expression of human TNF-α produced by PBMC. Thus, if there is a stimulation of the production of human TNF-α, this stimulation is a non-specific effect. Figure 2 shows the production of human TNF-α as a non-specific response of the addition of unmodified or modified siRNAs. In this figure it is clear that OMe-Prop produces a reduction from 20 to 50% of the immunostimulation activity, compared with w.m., at a concentration of 10 nM. These data confirm that the modifications developed in this invention provoke one non-specific response much smaller than the siRNAs without modification. Therefore, it is demonstrated that the biological effects observed are specific of the inhibition of TNF-α and not a general immune response induced by the siRNAs.

### EXAMPLE 3: Inhibition of TNF-α in vivo.

The therapeutic impact was assessed in a model of inflammatory bowel disease in mice by administering the compounds and their controls directly into the distal colon of diseased animals (rectal administration). Firstly, we tested the inhibitory efficacy in this animal model by measuring the TNF-α messenger RNA (mRNA) by the method of the reverse transcriptase and quantitative PCR, directly on the messenger RNA extracts of colon.

### Colitis model induced by 5% dextran sulfate sodium (DSS) in C57BI/6 mice

Experiments were performed using female mice from the C57BI/6 strain (Harlan Iberica). The animals were cared for under standard conditions at a temperature of 22° C and relative humidity of 70-80%, in 12 hours light / darkness cycles.

All protocols used with animals were approved by the ethical Committee of the Autonomous University of Barcelona and the animals were treated according to the European regulations.

The 5% DSS solution was added as drinking water to 10 weeks old mice. 6 animals were used as healthy controls and they did not receive treatment with DSS. 24 animals received treatment with DSS (MP Biomedicals; 36-50 kD molecular weight) in the drinking water.

During the experiment, all animals had water and food *ad libitum* (A04 chow, PanLab). The weight of the animals, food and water consumption were controlled daily in each cage.

### Assessment of the disease activity index (DAI)

During the period of the experiment, the animals were examined daily for the status of the following variables: (i) general condition of the animal, (ii) appearance of hair, (iii) presence and appearance of blood in the feces (benzidine method TMB), (iv) food and drink intake (grams or ml per day).

The DAI parameter is well correlated with histopathologic assessment of inflammation in the intestinal invaginations. The DAI is obtained from the following weighting: % increase in weight with respect to day 1 (PC), from 0 to 4 points; consistency of feces, from 0 to 4 points; appearance of the animal, from 0 to 3 points; blood in feces, from 0 to 3 points.

### Treatment protocol with compounds and their controls on the model of colitis

The compounds and their controls were suspended in RNAse-free water and they were mixed with Lipofectamine 2000 (Invitrogen, CA, USA). The complex was administered in the rectum of anesthetized mice (4 nanomoles per mouse).

### Anesthesia and euthanasia

A mixture of ketamine and xylazine (Imalgene 1000, Merial labs, Rompun 2%, Bayer) was injected intraperitoneally at doses of 55:15 mg/kg, diluted in saline buffer. The animals were sacrificed with an overdose of halothane and the blood was extracted via cardiac puncture. Then, different organs were extracted, which were frozen for further analysis.

### Activity of the enzyme Myeloperoxydase (MPO)

MPO is located in intracellular granules of polymorphonuclear neutrophils (PMNN) and it catalyzes the conversion of hydrogen peroxyde to water and ion oxygen. The activity of MPO is a quantitative indication of the degree of infiltration of leukocytes into inflamed tissue. O-dianisidine (Sigma) is used as a chromogen for the measurement of the activity of MPO. One MPO activity unit is the amount of enzyme that transforms 1 micromole of hydrogen peroxyde in water, in one minute.

### Histology

Colon samples from animals treated for histology were washed with phosphate buffered saline. To preserve the morphology and composition of the tissues they were fixed with 4% paraformaldehyde and then dehydrated through an ethanol gradient. The tissue was fixed in paraffin in the presence of toluene. The paraffin block was cut into sections of 8 micrometers thick, which were stained with hematoxylin / eosin.

### Statistics

All the data obtained were represented and were statistically analyzed with a software package (GraphPad Prism version 5.0 for Windows). The treatment groups were analyzed using the ANOVA method of a factor and subsequent non-parametric test (Newman-Koels). * < 0.05%, ** < 0.01% and *** < 0.001%.

### Analysis of the levels of production of TNF-α messenger RNA in the colon of mice by quantitative PCR.

For both cell and tissue total RNA purification, the RNeasy Mini Kit (Qiagen) was used according to the instructions of the manufacturer and for obtaining template DNA (copy DNA (cDNA) of the mRNA) it was carried out the reverse transcription of mRNA, using commercial equipment *Redy-To-Go You-Prime First-Strand Beads* (Amersham Biosciences) following the instructions of the manufacturer. The cDNA obtained from the samples to be studied, is used as template to be amplified by quantitative PCR. The Taqman probe test number Mm00443258_m1 (*Assay on Demand Gene Expression*) has been used to detect the modulation of the TNF-α messenger in the *ABI Prism 7700 Sequence Detection System* from Applied Biosystems.

Figure 3 shows that the disease model induced by administration of a 5% DSS solution produces an increase of the levels of TNF-α 3 days after the administration. A great increase in TNF-α in the colon of mice that have been treated with DSS and Scr can be observed, in comparison with those that have not been treated with DSS (healthy).

Under the selected experimental conditions (C57BI/6 mice, 5% DSS with 36-50 KDa molecular weight for 3 days), the treatment with the OMe-Prop compound administered 56 hours after the beginning of treatment with 5% DSS solution, reduced the levels of TNF-α messenger RNA by 40%, demonstrating that modified siRNA is effective in the model of colitis by rectal administration.

Figure 4 shows that the compounds of the invention are capable of stopping the development of the disease as a result of the decreased levels of TNF-α mRNA. Clinical disease parameters were analyzed 8 days after starting treatment with DSS. Mice were treated with compounds and their controls on days 2 and 4, using 4 nanomoles in each of the treatments. Figure 4 shows that the treatment with OMe-Prop produces a significant improvement both qualitative and quantitative on the clinical parameters of the colitis in the treated animals, compared to animals treated with w.m. or with Scr. The length of the colon (Figure 4D), the weight of the colon (Figures 4A and 4E) and the colon length/weight ratio (Figure 4F) are significantly better in animals treated with OMe-Prop. The disease activity index (DAI) (figures 4B and 4C) indicates an improvement in the animals treated with OMe-Prop. Also, the histological assessment shows significant beneficial impact in animals treated with OMe-Prop. The degree of inflammation as determined by the activity of MPO (Figure 4G) in treated colon extracts also shows a significant reduction after treatment with OMe-Prop. But the most important parameter, which is the degree of survival, is also clearly better in animals treated with OMe-Prop, showing that the beneficial effects seen locally in the degree of inflammation of the colon translate into a significant improvement of the general physiological condition of the animal.

## Claims

1. A compound of formula siRNA-P-O-R wherein:
i) siRNA is a small interfering RNA comprising at least one 2'-O-methyl modification;
ii) -P-O- represents a phosphodiester bond binding R in position 3' of either of the guide and partner strands of the siRNA; and
iii) wherein R is a radical which is selected from among: a C₁-C₆ alkyl substituted with at least one -OH group; a C₄-C₆ cycloalkyl substituted with at least one -OH group; a C₄-C₆ heterocycloalkyl; a heteroalkyl chain having between 2 and 7 atoms, wherein at least one of the atoms of the chain is an oxygen atom or a sulphur atom, the chain preferably being substituted with at least one -OH group; and a heteroaryl the ring whereof being formed of between 3 and 6 atoms and at least one of said atoms is an oxygen atom.

2. The compound according to the previous claim, wherein the guide strand of the siRNA of the compound of the invention inhibits or silences, totally or partially, the expression of a pro-inflammatory molecule.

3. The compound according to the previous claim, wherein the guide strand inhibits or silences the expression of at least one of the genes from the list comprising: tumour necrosis factor alpha (TNF-α); interleukin 1 beta; interleukin 6; and interleukin 8.

4. The compound according to any of the two previous claims, wherein the guide strand inhibits or silences the expression of the TNF-α or the interleukin 1 beta gene.

5. The compound according to any of the three previous claims, wherein the guide strand inhibits or silences the expression of the TNF-α gene.

6. The compound according to any of the previous claims, wherein R is a C₁-C₆ alkyl substituted with at least one -OH group.

7. The compound according to the previous claim, wherein R is a C₂-C₄ alkyl substituted with one or two -OH groups.

8. The compound according to any of the previous claims, wherein the phosphodiester bond binds a molecule of 1,2-propanediol or 1,3-propanediol, preferably 1,3-propanediol, in position 3' of any of the siRNA strands.

9. The compound according to any of claims 1 to 5, wherein R is a C₄-C₆ cycloalkyl substituted with at least one -OH group.

10. The compound according to the previous claim, wherein the phosphodiester bond binds a molecule of 4-cyclohexanediol in position 3' of any of the siRNA strands.

11. The compound according to any of claims 1 to 5, wherein R is a C₄-C₆ heterocycloalkyl, preferably substituted with at least one -OH group.

12. The compound according to the previous claim, wherein R is a oxetanyl, aziridinyl, azetidinyl, tetrahydrofuranyl, pyrrolidinyl, morpholinyl, dithianyl, thiomorpholinyl, piperidinyl, tetrahydropyranyl, piperazinyl or trithianyl.

13. The compound according to any of claims 1 to 5, wherein R is a heteroalkyl chain having between 2 and 7 atoms, wherein at least one of the atoms of the chain is an oxygen atom or a sulphur atom.

14. The compound according to the previous claim, wherein R is a heteroalkyl chain having between 4 and 6 atoms wherein at least one of the atoms of the chain is an oxygen atom or a sulphur atom.

15. The compound according to the previous claim, wherein the phosphodiester bond binds a molecule of diethylene glycol or diethylene glycol thioether in position 3' of any of the siRNA strands.

16. The compound according to any of claims 1 to 5, wherein R is a heteroaryl formed by between 3 and 6 atoms and at least one of said atoms is an oxygen atom.

17. The compound according to any of the previous claims, wherein R is linked through a phosphodiester bond to the position 3' of the partner strand.

18. The compound according to any of the previous claims, wherein between 1 and 7 nucleotides that form the siRNA comprise a 2'-O-methyl modification.

19. The compound according to any of the previous claims, wherein the nucleotides forming the siRNA comprising the 2'-O-methyl modification are on the 5' end.

20. The compound according to any of the previous claims, wherein the nucleotides forming the siRNA comprising modifications 2'-O-methyl are on the partner strand.

21. The compound according to any of the previous claims, wherein at least one nucleotide forming the siRNA is a deoxyribonucleotide.

22. The compound according to the previous claim, wherein at least one nucleotide of the 3' end of the siRNA is a deoxyribonucleotide.

23. The compound according to any of the two previous claims, wherein the deoxyribonucleotides of the 3' end of the siRNA are thymidine deoxyribonucleotides.

24. The compound according to any of the three previous claims, wherein the deoxyribonucleotides are at the 3' end of the partner strand of the siRNA.

25. The compound according to any of the previous claims, wherein each strand of the siRNA comprises between 15 and 40 nucleotides.

26. The compound according to any of the two previous claims, wherein the sequence of the partner strand of the siRNA is SEQ ID NO: 1.

27. The compound according to any of the three previous claims, wherein the sequence of the guide strand of the siRNA is SEQ ID NO: 5.

28. The compound according to any of the four previous claims, wherein the sequence of the partner strand of the siRNA is SEQ ID NO: 1 and the sequence of the guide strand of the siRNA is SEQ ID NO: 5.

29. A pharmaceutical composition comprising the compound according to any of the previous claims.

30. The pharmaceutical composition according to the previous claim, also comprising a pharmaceutically acceptable vehicle or diluent.

31. The pharmaceutical composition according to the previous claim, wherein the vehicle or diluent is a transfection agent.

32. The pharmaceutical composition according to the previous claim, wherein the transfection agent is selected from the list comprising polyethylene glycol, cholesterol, polyethylenimide (PEI), cell penetrating peptides, oligoarginine, poly-lysine, rabies virus glycoprotein, gold nanoparticles, dendrimers, carbon nanotubes and lipids.

33. The pharmaceutical composition according to the previous claim, wherein the transfection agent is a lipid.

34. The pharmaceutical composition according to the previous claim, wherein the lipid is a cationic lipid.

35. The pharmaceutical composition according to the previous claim, wherein the lipid is a liposome.

36. The pharmaceutical composition according to any of the seven previous claims, also comprising another biologically active substance.

37. Use of the compound according to any of claims 1 to 28 or of the pharmaceutical composition according to any of claims 29 to 36 for the preparation of a medicine.

38. Use according to the previous claim for the treatment of an inflammatory disease.

39. Use according to the previous claim for the treatment of a disease that is selected from the list comprising: inflammatory bowel disease, rheumatoid arthritis, osteoarthritis and chronic obstructive pulmonary disease.

40. Use according to the previous claim wherein the inflammatory disease is inflammatory bowel disease.


SEQUENCE LISTING

<110>  Consejo Superior de Investigaciones Científicas(CSIC).
       Universidad Autónoma de Barcelona
       Universidad de Barcelona

<120>  DERIVATIVES OF SMALL INTERFERING RNAS AND USE THEREOF

<130>  ES1641.707

<160>  5

<170>  PatentIn version 3.5

<210>  1
<211>  21
<212>  DNA
<213>  Mus musculus


<220>
<221>  modified_base
<222>  (1)..(1)
<223>  gm

<220>
<221>  misc_feature
<222>  (1)..(2)
<223>  n is a, c, g, t or u

<220>
<221>  modified_base
<222>  (2)..(2)
<223>  um

<400>  1
nngccuaugu cucagccuct t                                                21


<210>  2
<211>  21
<212>  DNA
<213>  Mus musculus

<400>  2
gugccuaugu cucagccuct t                                                21


<210>  3
<211>  21
<212>  DNA
<213>  Artificial Sequence

<220>
<223>  Negative control partner strand of the siRNA.


<220>
<221>  modified_base
<222>  (1)..(1)
<223>  cm

<220>
<221>  misc_feature
<222>  (1)..(2)
<223>  n is a, c, g, t or u

<220>
<221>  modified_base
<222>  (2)..(2)

<223>  2'-O-methyl-adenosine

<400>  3
nngucgcguu ugcgacuggt t                                                21


<210>  4
<211>  21
<212>  DNA
<213>  Artificial Sequence

<220>
<223>  Negative control guide strand of the siRNA.

<400>  4
ccagucgcaa acgcgacugt t                                                21


<210>  5
<211>  21
<212>  DNA
<213>  Mus musculus

<400>  5
gaggcugaga cauaggcact t                                                21